# FASCICULE DE BREVET EUROPEEN

(11) **EP 1 499 330 B1**
(45) Date de publication et mention de la délivrance du brevet: **25.11.2009**
(21) Numéro de dépôt: 03740670.9
(22) Date de dépôt: 25.04.2003
(51) Int. Cl.: A61K 33/36, A61P 37/00

(54) **THERAPIE PAR L'ARSENIC DE MALADIES AUTOIMMUNES**
ARSENTHERAPIE ZUR BEHANDLUNG VON AUTOIMMUNERKRANKUNGEN
ARSENIC THERAPY FOR AUTOIMMUNE DISEASES

(30) Priorité: 26.04.2002 FR 0205276
(43) Date de publication de la demande: 26.01.2005
(73) Titulaire: CENTRE NATIONAL DE LA RECHERCHE SCIENTIFIQUE (CNRS), 75016 Paris (FR)
(72) Inventeur: CHELBI ALIX, Kmar, F-75011 Paris (FR); BOBE, Pedro, F-75006 Paris (FR)
(74) Mandataire: Warcoin, Jacques
(86) Numéro de dépôt international: PCT/FR2003/001314
(87) Numéro de publication internationale: WO 2003/090766

(56) Documents cités:
- EP-A- 0 804 928
- WO-A-00/07616
- H BURKHART ET ALL.: "Xenobiotic immunosuppresive agents: therapeutic effects in animal models of autoimmune diseases" RHEUMATOLOGY INTERNATIONAL, vol. 17, no. 3, 1997, pages 85-90, XP002224717

## Description

L'invention concerne l'utilisation de l'arsenic pour le traitement et/ou la prévention de maladies auto-immunes, notamment du syndrome auto-immun lymphoprolifératif.
On connaît déjà l'utilisation d'arsenic pour traiter différents types de cancers : leucémie, lymphome, cancer des ovaires, cancer du poumon.
De tels traitements sont décrits dans les documents FR 2 782 010, US 6 333 028, FR 2 539 993, US 6 191 123, EP 955 052, WO 9924029, WO 9918798, WO 9955344. On connaît également l'utilisation de l'arsenic pour traiter d'autres maladies tels que des coccidioses, des maladies parasitaires, des dermatomycoses, le syndrome d'asthénie chronique. Ces traitements sont notamment décrits dans les documents FR 2 572 730, US 5 550 153, WO 9421249, WO 9501789.
Mais l'utilisation d'arsenic pour le traitement de maladies auto-immunes n'est jusqu'à présent pas connu.

Par auto-immunité, on entend un état d'immunisation d'un individu contre lui-même. Les maladies auto-immunes sont des maladies du système immunitaires caractérisées par la production d'anticorps (appelé auto-anticorps) qui réagissent avec des antigènes (appelé auto-antigène) provenant des tissus du propre patient (pour revue voir Schwartz *et al* (1984)). Les maladies auto-immunes les plus courantes sont : le lupus systémique érythémateux, le lupus érythéimteux aigu disséminé, l'uvéite, la maladie de Bechet, la sarcoïdose, le syndrome de Sjôgren, la polyarthrite rhumatoïde, la polyarthrite juvénile, le syndrome de Fiessinger-Leroy-Reiter, la goutte, l'ostéoarthrose, la polymyosite, la myocardite, la cirrhose biliaire primitive, la maladie de Crohn, la colite ulcéreuse, la sclérose en plaques et autres maladies démyélinisantes, l'anémie aplasique, le purpura thrombocytopénique essentiel, toute maladie associée à une lymphoprolifération non tumorale, le lymphome à lymphocytes B, le panhypopituitarisme de Simmonds, la maladie de Basedow-Graves et l'ophtalmopathie de Graves, la thyroïdite subaiguë et la maladie de Hashimoto, la maladie d'Addison, les hépatites chroniques, le diabète sucré insulino-dépendant (type 1).
Les médicaments de l'art antérieur utilisés pour le traitement de maladies auto-immunes, en particulier les maladies systémiques, entraînent généralement une suppression non spécifique générale du système immunitaire. Le plus souvent, ces médicaments ne distinguent pas la réponse pathologique immunitaire et une réponse immunitaire protectrice de l'organisme. Ainsi, des médicaments immuno suppresseurs (exemple : corticostéroïde, azathioprine, cyclophosphamide et cyclosporine) sont souvent administrés pour supprimer la prolifération des lymphocytes auto-réactifs. Des médicaments anti-inflammatoires sont souvent prescrits à des patients atteints d'arthrite rhumatoïde. De façon globale, ces médicaments présentent des effets secondaires néfastes, la suppression de la réponse immunitaire entraînant des risques d'infection et de cancer.

La demande de brevet européen EP 0 804 928 (Vorobieva, Tamara Vasilievna) décrit l'utilisation d'un composé choisi parmi le dichlorure de mercure, l'arsénite de potassium et l'arséniate de sodium, à des fins d'immunomodulation.

Dans la mesure où l'art antérieur est extrêmement riche en substances candidates susceptibles d'agir contre de telles maladies, il est très difficile de repérer des substances réellement actives *in vivo* et capables de traiter les maladies auto-immunes.
L'invention vise à pallier les inconvénients de l'art antérieur en proposant des composés efficaces pour le traitement et/ou la prévention de maladies auto-immunes, avec des effets secondaires non souhaités très réduits ou totalement absents.
A cet effet l'invention a pour objet selon un premier aspect l'utilisation d'un composé arsenic choisi parmi : As₂O₃, As₄O₆, As₂S₂, As₂S₃, As₂S₅, As₄S₄, tout particulièrement As₂O₃, pour la préparation d'un médicament pour le traitement et/ou la prévention de maladies auto-immunes.
En particulier, on ciblera les maladies choisies mentionnées précédemment.
De manière préférée on ciblera des maladies auto-immunes appartenant à certaines catégories de trouble métabolique, telles que :
- un défaut de maintien de l'homéostasie des organes lymphoïdes, type ALPS ;
- un défaut de maintien de l'homéostasie du foie (hépatomégalie) ;
- des maladies cutanées (lupus, psioriasis, ...) ;
- une apoptose défectueuse de cellules proliférantes du foie, de la peau,
- une apoptose défectueuse de cellules proliférantes du système immunitaire pouvant se traduire entre autre par une surexpression de FasL (hépatites chroniques auto-immunes...) ou une production anormale de cytokines (polyarthrite rhumatoïde, diabète sucré insulo-dépendant (auto-immun)...).
Les produits de la présente invention peuvent également être utilisés pour le traitement de la réaction du greffon contre l'hôte.
Selon des modes de réalisation particulièrement préférés, les maladies à traiter sont le Syndrome auto-immun lymphoprolifératif (ALPS), le Lupus érythémateux, la polyarthrite rhumatoïde, le syndrome de Sjörgen, les hépatites chroniques auto-immunes, le diabète sucré insulo-dépendant.
Dans la présente demande, on utilisera le terme « arsenic », ou « composé arsenic », ou « dérivé d'arsenic ». Ce terme englobe les différentes formes d'arsenic précédemment cités, qui ont un effet préventif ou curatif contre les pathologies cibles. La présente demande décrit en outre des tests *in vivo* qui permettent de cribler les composés efficaces, qui présentent des résultats d'activité biologique d'au moins 10, 20, 50, 80, 90, 100, 150, 200 % de ceux obtenus dans la description détaillée qui suit utilisant l'arsenic As₂O₃.
L'homme du métier disposant de ces tests peut donc sans effort excessif identifier des composés arsenic thérapeutiquement efficaces <choisi parmi : As₂O₃, As₄O₆, As₂S₂, As₂S₃. As₂S₅, As₄S₄, tout particulièrement As₂O₃,> Les composés arsenic candidats peuvent être présélectionnés à l'aide de tests *in vitro* appropriés avant d'être testés *in vivo.*
Ces composés arsenic peuvent être criblés afin de mesurer leur efficacité thérapeutique par un procédé de criblage *in vivo* comprenant l'administration d'un composé arsenic candidat à des animaux atteints d'une maladie auto-immune, typiquement à une dose quotidienne de l'ordre de 5 à 10 mg/Kg de poids corporel.
On décrit également une composition pharmaceutique destinée au traitement et/ou à la prévention d'au moins une maladie auto immune **caractérisée en ce qu**'elle comprend une quantité efficace d'un composé arsenic ou un sel pharmaceutiquement acceptable de ce composé. Cette quantité efficace destinée à être administrée quotidiennement est une dose de 3 à 10 mg/kg de poids corporel, de préférence 5 à 10 mg/kg.
Cette composition peut comprendre en outre un transporteur ou excipient pharmaceutiquement acceptable. Elle est typiquement sous une forme appropriée pour une administration orale ou parentérale.
Le terme sel pharmaceutiquement acceptable fait référence à des sels préparés à partir d'acides et de bases non toxiques pharmaceutiquement acceptables, incluant des acides et des bases inorganiques et organiques. On peut utiliser notamment un sel acide de sodium ou de potassium, un iodure d'arsenic.
Par ailleurs on décrit ici l'utilisation pour le traitement de maladies auto-immunes de composés autres que l'arsenic, mais capables tout comme l'arsenic d'inhiber des activités phosphatases.
Le transporteur peut être de type très varié, selon la forme de la préparation utilisée pour l'administration, notamment orale ou parentérale (pastille, capsule, poudre, injection intraveineuse, infusion).
Pour une administration orale, la préparation pharmaceutique peut être sous forme liquide, par exemple en solution, sous forme de sirop ou de suspension, ou sous forme de poudre destinée à être remise en solution. Une telle préparation liquide peut être préparée selon les techniques appropriées avec des excipients pharmaceutiquement acceptables tels que des agents de suspension (par exemple : sorbitol, dérivés de cellulose), des agents émulsifiants (par exemple: lécithine ou acacia), des transporteurs non aqueux (par exemple : huile végétale fractionnée), et des agents conservateurs (par exemple : acide sorbique).
Lorsqu'il s'agit de préparations orales solides (pastille, poudre, capsule, comprimé), les compositions pharmaceutiques sont préparées à l'aide d'excipients appropriés tels que des agents de liaison (amidon de maïs, polyvinyle pyrrolidone, hydroxypropile méthylcellulose), des agents de remplissage (lactose, cellulose, microcrystalline), des agents lubrifiants (magnésium stéarate, talc, silice), des agents de gonflement (sodium lauryl sulfate).
Pour une administration par inhalation, les composés sont typiquement inclus dans une préparation de type aérosol à l'aide de gaz approprié.
Les dérivés d'arsenic peuvent être également formulés pour une administration parentérale par exemple, par injection continue ou non. Les milieux liquides sont généralement analogues (eau, glycol, huile, tampon) à ceux utilisés pour des préparations orales.
Les formulations par injection peuvent se présenter sous forme d'unités de dosage (ampoules, mini containers) avec un agent de protection approprié. Ces compositions à injecter peuvent également être sous la forme de suspension, de solution ou d'émulsion et peuvent contenir des agents stabilisants et/ou des agents dispersants. Le principe actif peut également être préparé sous forme de poudre avec un transporteur approprié. On décrit également des nécessaires ou kits comprenant au moins un contenant renfermant le dérivé d'arsenic sous une forme pharmaceutiquement acceptable. Par exemple, l'arsenic peut être sous forme d'une solution pharmaceutiquement acceptable telle que une solution saline, une solution de dextrose, une solution tampon stérile, le kit peut également comprendre les moyens d'injection appropriés tels qu'une seringue emballée stérile.
La dose thérapeutique utilisée dans le traitement de maladies auto-immunes est variable selon la gravité et les conditions de traitement. La dose, le cas échéant la fréquence, sont à adapter en fonction notamment de l'âge, du poids corporel. Compte tenu de la toxicité potentielle de l'arsenic le dosage et la durée du traitement sont déterminés de manière appropriée, selon la gravité de la maladie et du caractère durable ou non de la guérison. Le traitement pouvant durer de quelques jours à quelques mois jusqu'à ce que la guérison totale ou au moins partielle soit atteinte. La formulation est typiquement administrée quotidiennement, pendant une période de 10 à 50 jours. On pourra effectuer plusieurs traitements successifs, espacés de l'ordre d'un mois.
En outre, l'arsenic peut être administré le cas échéant avec d'autres principes actifs contribuant au traitement des maladies ciblées. Ainsi on peut citer l'utilisation de l'arsenic en association avec des corticostéroïdes comme la Prednisone ou des drogues comme le Methotrexate pour le traitement de syndromes auto-immuns et/ou de pathologies inflammatoires chroniques.
On divulgue en outre, une méthode de tracement de maladies auto-immunes, notamment chez l'homme, comprenant l'administration au patient d'une quantité pharmaceutiquement efficace d'un dérivé d'arsenic. L'invention concerne une méthode de traitement comprenant l'administration de 5 à 10 mg d'arsenic par kilogramme de poids corporel par jour. L'administration est typiquement orale ou parentérale.
L'effet du traitement avec les composés arsenic sur les maladies auto-immunes étudiées peut être suivi par des techniques appropriées telles que décrites dans les exemples ci-après, notamment mesure du poids des organes (ganglions, rate ...), étude de la structure des tissus (étude externe, anatomopathologie). Par traitement on entend un traitement curatif, ou préventif. Les inventeurs ont démontré l'efficacité des composés arsenic pour prévenir l'apparition de maladies auto-immunes comme cela est décrit dans les exemples plus loin.
D'autres objets et avantages de l'invention apparaîtront à la lecture de la description détaillée illustrée par les dessins dans lesquels :
- les figures 1A et 1B présentent la guérison obtenue grâce à l'arsenic sur des souris MRL/lpr, respectivement la morphologie externe et l'étude de la peau du cou par anatomopathologie ;
- la figure 2 présente l'effet de l'arsenic sur des souris MRL/lpr, souris non traitée à gauche ayant une exophtalmie, souris traitée à droite totalement guérie ;
- les figures 3 présentent des coupes de tissu pulmonaire avec de fortes infiltrations par des cellules lymphoïdes autour des vaisseaux et bronchioles (contrôle) et une architecture pulmonaire normale (Arsenic)
- Les figures 4A et 4B illustrent le retour à l'anatomie normale des organes internes grâce au traitement à l'arsenic ;
- la figure 5 représente la diminution du pourcentage de lymphocytes T CD4-CD8-B220+ grâce à l'arsenic ;
- les figures 6 présentent la réduction du taux de cytokines et du taux de FasL membranaire grâce à l'arsenic
- les figures 7 présentent la réduction, grâce à l'arsenic, du taux d'autoanticorps sous forme de complexes immuns, accumulés dans le rein, et l'infiltration du rein par des cellules du système immunitaire chez les animaux non traités à l'arsenic.
- la figure 8 illustre la disparition du taux anormal des anticorps anti-ADN grâce à l'arsenic : les anticorps anti-ADN sont une caractéristique de Lupus chez l'homme et la souris MRL/lpr;
- la figure 9 illustre la survie des animaux: tous les animaux témoins sont morts au bout de 4 mois, tous les animaux traités sont vivants.

Les inventeurs ont travaillé sur des souris dites MRL homozygotes pour la mutation lpr (MRL/lpr). La lymphoprolifération (*lpr*) se traduit par une accumulation de lymphocytes T activés, de phénotype CD4⁺CD8⁻ ou CD4⁻CD8⁺, qui ont échappé au processus de régulation négative par la voie d'apoptose. Ainsi on aboutit chez la souris MRL/lpr à une hypertrophie des ganglions lymphatiques dont la masse est 100 fois supérieure à celle des ganglions lymphatiques de souris MRL+/+, congéniques pour le gène *lpr*, à une rate dont la taille est multipliée par 7, et un thymus dont le poids est à la 22^{ème} semaine le double de celui des souris MRL+/+. Ces lymphocytes T activés qui s'accumulent vont progressivement réprimer l'expression de leurs récepteurs CD4 ou CD8 et devenir des lymphocytes T double négatifs, de phénotype : TCRαβICD4⁻CD8⁻B220⁺, qui seront responsables de la lymphoprolifération.

### La souris MRL/lpr est un modèle spontané de pathologies auto-immunes.

La souris MRL/lpr est en particulier un modèle, spontané, de maladies auto-immunes comme le lupus érythémateux disséminé avec la présence de lésions érythémateuses de la peau et de polyarthrite rhumatoïde. Cet animal présente également un syndrome de Sjögren caractérisé par la destruction des glandes salivaires et lacrymales, suite à une infiltration de cellules lymphoïdes. Ces souris produisent, au cours de leur vieillissement, de grande quantité d'auto-anticorps qui sont responsables d'une glomérulonéphrite mortelle par dépôts d'immuns complexes au niveau des glomérules du rein. Ainsi, la durée de vie de la MRL/lpr est considérablement raccourcie par rapport à celle de la MRL+/+ : ∼4-5 mois chez la souris MRL/lpr par rapport à ∼2 ans chez la souris MRL+/+. La souris congénique MRL+/+ développe une forme atténuée de glomérulonéphrite mais ne présente pas de lymphoprolifération, ainsi la mutation lpr agirait comme facteur d'accélération dans la survenue du syndrome lupique.

### Thérapie par l'arsenic des pathologies de la souris MRL/lpr

Dans un premier temps, les inventeurs ont déterminé la dose optimum d'arsenic (As₂O₃) à injecter aux MRL/lpr. Deux concentrations ont été choisies : 1 et 5µg d'As₂O₃/g de souris. A la date T0, ces injections ont été réalisées quotidiennement. Le groupe à la plus forte dose a montré au bout d'une semaine de traitement des signes spectaculaires d'amélioration des lésions cutanées spécifiques du lupus, ce qui n'était pas le cas pour les souris traités à la plus faible dose. Sachant que les souris auto-immunes MRL/lpr meurent en moyenne en 4 à 5 mois, comparé aux souris normales MRL+/+ qui meurent en ∼2 ans, les inventeurs ont décidé de ne conserver pour les traitements ultérieurs que la dose de 5µg/g de souris. Les souris MRL+/+ témoins ayant reçu cette dose d'arsenic ne présentent à T0+5 mois, aucun signe de pathologies liées au traitement.

Les inventeurs ont dès la fin de la première semaine de traitement, constitué 5 groupes d'animaux :
- 1^{er} groupe, vise au traitement curatif d'une pathologie déjà présente,
- 2^{ème} groupe, vise à prévenir l'apparition des pathologies,
- 3^{ème} groupe, vise à déterminer la durée de vie des animaux traités,
- 4^{ème} groupe, vise à déterminer l'effet dose,
- 5^{ème} groupe, vise à évaluer l'effet d'un arrêt du traitement.

Chacun des cinq groupes possède ses témoins consistant à injecter le tampon de dilution de l'arsenic. De plus, les animaux présents dans les groupes témoins et traités sont issus de la même portée, ce qui permet d'obtenir des comparaisons fiables. A T0+5mois, plus de 60 souris (MRL/lpr et MRL+/+) sont en traitement.

### Résultats du groupe 1

Cette étude vise au traitement curatif d'une pathologie déjà présente.

### A) Etude des lésions cutanées

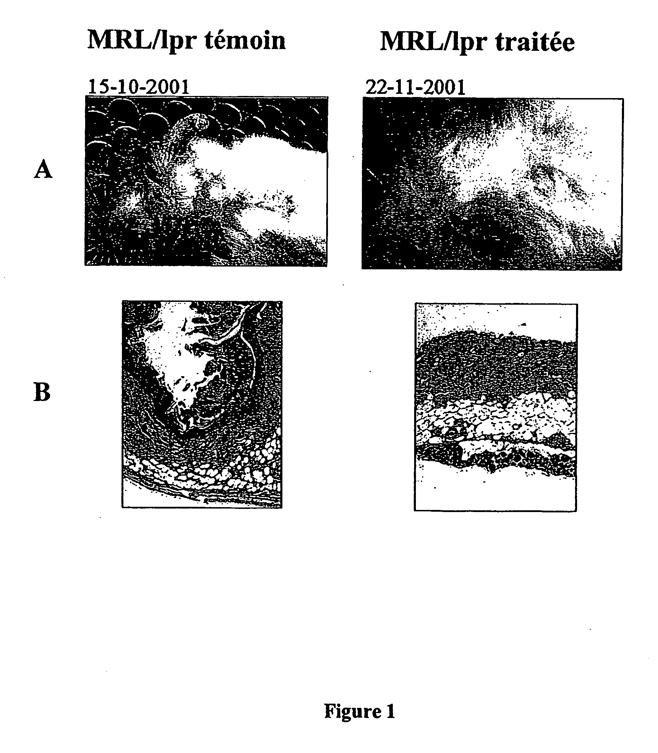

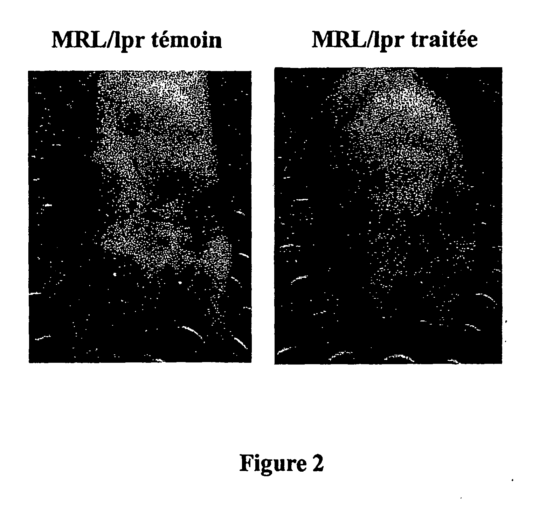

Dès la fin du premier mois de traitement, les souris MRL/lpr présentaient une disparition complète des lésions cutanées liées au lupus (Figure 1) et une réduction spectaculaire de l'exophtalmie caractéristique de la pathologie de la souris MRL/lpr (Figure 2). Depuis cette date, d'autres groupes traités et non traités ont été constitués et les groupes traités par l'As₂O₃ présentent systématiquement une disparition de l'exophtalmie et des atteintes cutanées. De plus, par des études anatomo-pathologiques de la peau (Figure 1B) la disparition totale du tissu pathologique a été confirmée chez les souris MRL/lpr traités par l'As₂O₃ mais pas chez les animaux MRL/lpr contrôles. Ce traitement par l'arsenic est donc efficace pour le traitement des lésions cutanées liées à une pathologie auto-immune comme le lupus.

Parallèlement à ces études sur la peau, d'autres organes cibles de pathologies auto-immunes comme le rein, le poumon, le foie, l'oeil, etc...., ont été prélevés et analysés par anatomopathologie. De plus, ces différents organes ainsi que la rate, les ganglions et le thymus ont été pesés. Les résultats sont présentés dans le tableau 1. En résumé, le traitement par l'arsenic ramène à un poids normal, comparable à celui des organes de la MRL++, les organes suivants : ganglions, rate et foie. Il est à noter que l'action de cet agent est ciblée sur les organes lymphoïdes et le foie, sachant que la MRL/lpr présente des anomalies d'homéostasie des organes lymphoïdes et du foie.

**Tableau 1 : Poids des organes en grammes**

| | MRL+/+ témoin | MRL+/+ Arsenic | MRL+/+ Arsenic | MRL/lpr témoin | MRL/lpr Arsenic | MRL/lpr Arsenic | MRL/lpr * Arrêt de traitement | MRL/lpr * Arrêt de traitement |
|---|---|---|---|---|---|---|---|---|
| Ganglions axillaires | 0,01 | **0,01** | **0,01** | 0,38 | **0,01** | **0,02** | 0,38 | 0,15 |
| Ganglions mésentériques | 0,03 | **0,05** | **0,03** | 2,61 | **0,05** | **0,05** | 1,97 | 1,03 |
| Rate | 0,09 | **0,10** | **0,15** | 0,53 | **0,13** | **0,17** | 0,41 | 0,20 |
| Foie | 2,46 | **2,40** | **2,45** | 3,30 | **2,58** | **2,89** | 3,21 | 3,12 |
| 2 poumons | 0,35 | **0,32** | **0,30** | 0,69 | **0,33** | **0,40** | 0,51 | 0,50 |
| Rein | 0,47 | **0,44** | **0,44** | 0,44 | **0,52** | **0,53** | 0,55 | 0,55 |
| Coeur | 0,21 | **0,22** | **0,22** | 0,29 | **0,20** | **0,25** | 0,29 | 0,26 |

| | | | | | | | | |
|---|---|---|---|---|---|---|---|---|
| * Arrêt de traitement : souris MRL/lpr traitée à l'arsenic pendant deux mois. Puis arrêt du traitement pendant une période de deux mois avant de sacrifier l'animal. | | | | | | | | |

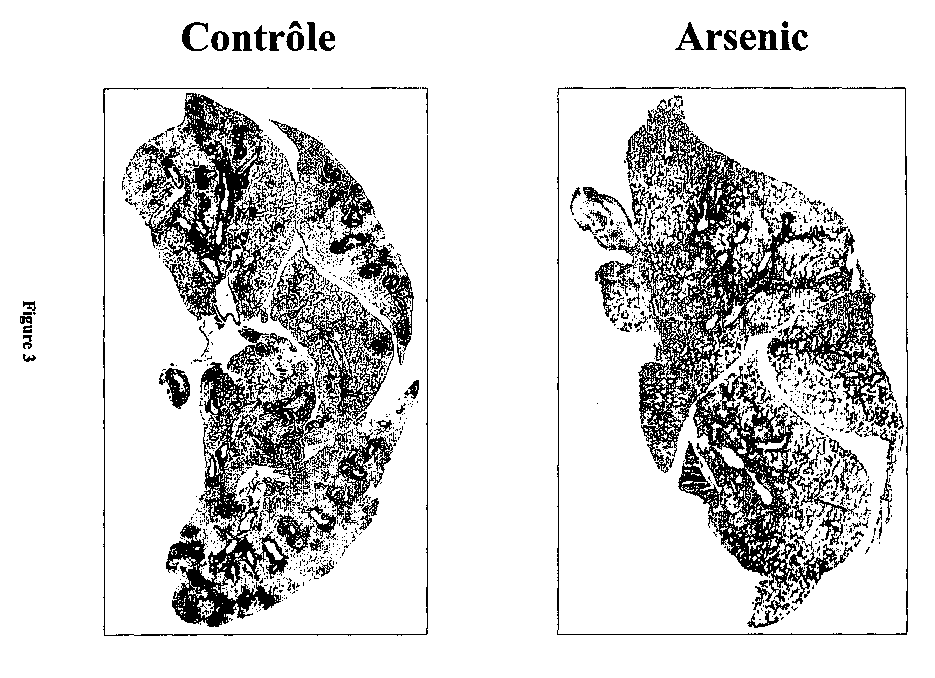

De plus, il est à noter que dans les formes sévères de Lupus Erythémateux Disséminé, les patients présentent une inflammation importante du poumon avec de fortes infiltrations par des cellules lymphoïdes. Les souris auto-immunes MRL/lpr présentent les mêmes symptômes avec, autour des vaisseaux et des bronchioles, de fortes infiltrations par des cellules lymphoïdes (figure 3A, contrôle). En revanche, les souris traitées par As2O3 ont une architecture pulmonaire normale avec seulement quelques cellules lymphoïdes autour des vaisseaux et aucune autour des bronchioles (figure 3A, arsenic)

### B) Etude de la lymphoprolifération

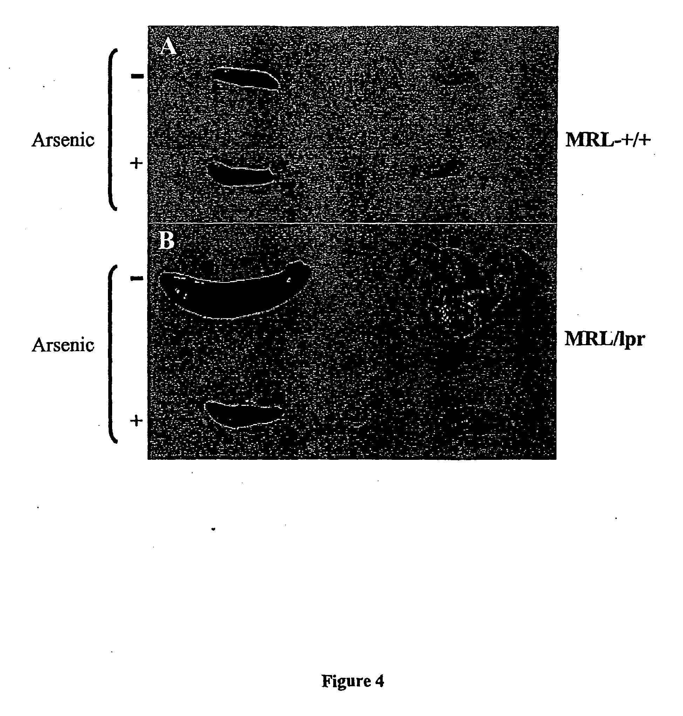

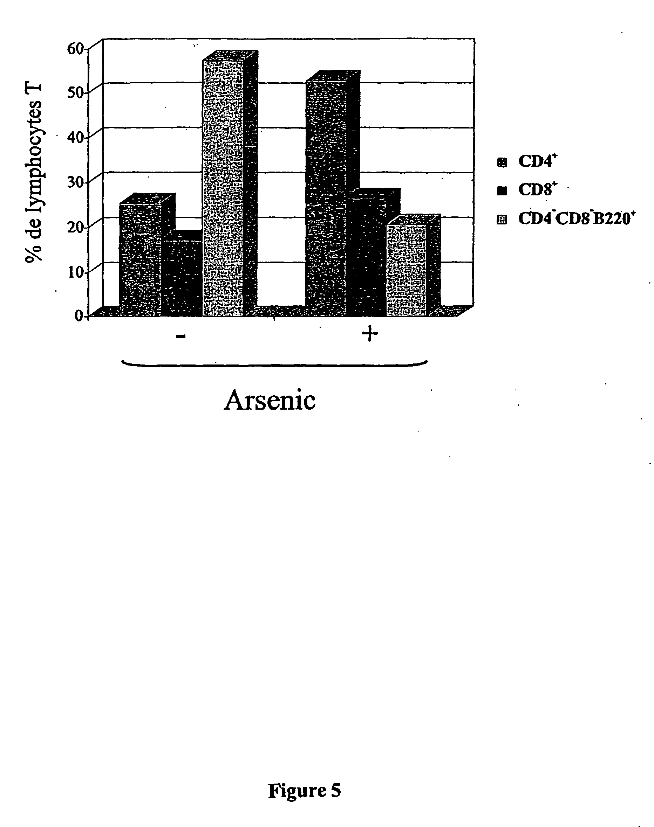

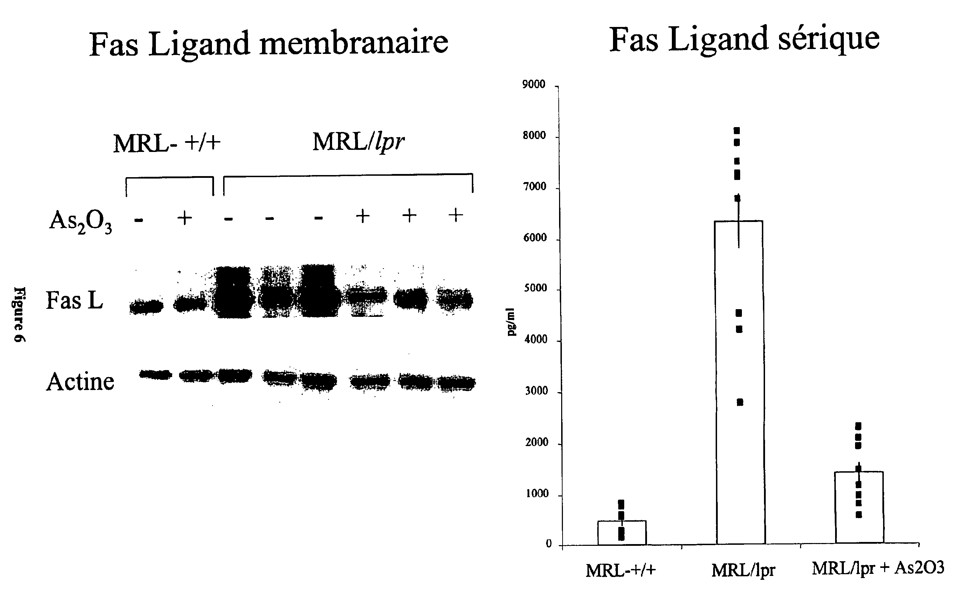

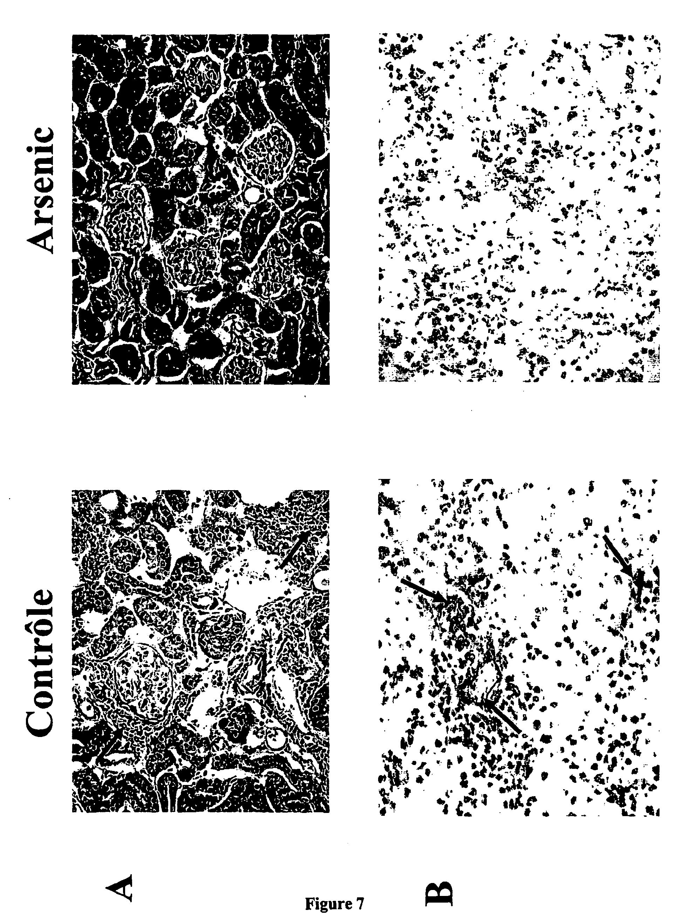

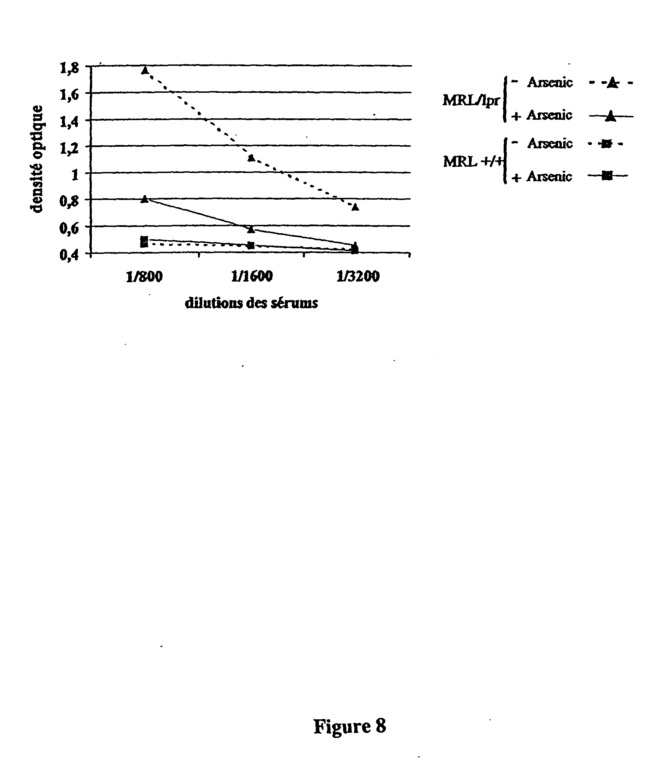

Après 1 mois et 20 jours de traitement un groupe de 3 souris MRL/lpr témoins et un groupe de 3 MRL/lpr traitées ont été sacrifiés afin d'évaluer l'efficacité du traitement sur la lymphoprolifération. Les organes analysés sont le thymus, la rate et les ganglions lymphatiques. La figure 4, illustre la diminution spectaculaire de la taille de la rate et de celle d'un des ganglions mésentériques. Le phénotype des populations lymphoïdes présentes dans cette rate et ce ganglion a été déterminé par cytométrie de flux. En résumé, le traitement par l'arsenic induit une disparition de la lymphoprolifération grâce à l'élimination quasi complète de la population de lymphocytes T double négatifs, de phénotype TCRαβCD4⁻CD8⁻B220⁺, responsables de la lymphoprolifération. De plus, cette population cellulaire de phénotype anormal est remplacée par des lymphocytes T de phénotype normal CD4⁺CD8⁻ et CD4⁻CD8⁺ (Figure 5). Cependant, le taux de réduction du nombre de ces cellules T doubles négatives est dépendant de la durée du traitement. Ainsi, un traitement de trois mois permet d'éliminer la totalité des cellules T doubles négatives.
En outre, les souris auto-immunes MRL/lpr présentent une production élevée de cytokines pro-inflammatoires et de cytokines de mort (death cytokines) telles que les IFN alpha et gamma, l'IL-18, le TNF-alpha et le FasL soluble, contrairement aux souris normales MRL+/+. Un traitement à l'arsenic de ces souris auto-immunes MRL/lpr ramène le taux de cytokines à un niveau normal comme celui observé chez les souris normales MRL+/+. Cette réduction du taux de cytokines et du taux de FasL membranaire soluble (figure 6) est liée à l'élimination des lymphocytes T activés, responsables de la lymphoprolifération.
De plus, le lupus Erythémateux Disséminé et la polyarthrite Rhumatoïdales sont caractérisés par la présence de taux élevés d'autoanticorps sériques. Chez les souris auto-immunes MRL/lpr, ces autoanticorps s'accumulent dans le rein sous forme de complexes immuns (figure 7, contrôle B, flèches) et sont responsables d'une glomérulonéphrite qui entraîne la mort des souris en 4 à 5 mois. Le traitement des souris MRL/lpr par de l'arsenic réduit de 60 à 70% le taux d'autoanticorps (IgG anti-ADN double brin). Cette diminution du taux d'autoanticorps a pour conséquence une absence de complexes immuns au niveau du rein (figure 7, arsenic B), une réduction significative de la glomérulonéphrite, et une augmentation de la survie. De plus, les souris traitées par l'arsenic ne présentent pas d'infiltration du rein par des cellules du système immunitaire (figure 7, Arsenic A) comparé aux animaux non traités (figure 7, contrôle A, flèches)
En conclusion, l'ensemble des résultats obtenus avec le groupe 1 montre que l'arsenic est un traitement efficace pour l'élimination des lymphocytes T activés responsables chez la souris MRL/lpr du syndrome auto-immun lymphoprolifératif (figure 8). En effet, l'arsenic est efficace pour le traitement des lésions cutanées liées au lupus érythémateux disséminé de la souris MRL/lpr. L'arsenic est également un traitement efficace pour la réduction du taux de cytokines chez la souris MRL/lpr, ainsi que pour le traitement de la glomérulonéphrite chez la souris MRL/lpr par réduction du taux d'auto-anticorps.

### B) Etude de l'évolution du taux de glucose

Des souris NOD (Non Obese Diabetic) femelles, âgées de 5 mois, et qui développaient les premiers symptômes du diabète, ont été traitées durant 2 mois par de l'arsenic. L'évolution de la pathologie est évaluée par la mesure de la quantité de glucose dans les urines. Seules les souris NOD traitées par l'arsenic présentent un taux normal de glucose dans les urines.

### Résultats du groupe 2

Cette étude vise à prévenir l'apparition des pathologies.

Ce groupe d'animaux, commencé à T0, à pour but d'évaluer l'effet préventif d'un traitement à l'arsenic sur le développement de la lymphoprolifération et des pathologies auto-immunes. Pour ce faire, 4 groupes d'animaux âgés de 1mois ½ ont été formés. Deux de ces groupes sont constitués de souris MRL/lpr et les deux autres de souris MRL+/+. Un groupe de MRL/lpr et un groupe de MRL+/+ ont été traités par 5µg d'arsenic par gramme de souris, les deux autres groupes témoins sont traités par le tampon de dilution de l'arsenic. A T0+1 mois, des animaux de chaque groupe ont été sacrifiés. Les organes analysés sont le thymus, la rate, les ganglions lymphatiques, la peau, le foie, le rein et le coeur. Une absence quasi complète de la lymphoprolifération a été observée chez les souris MRL/lpr traitées par l'arsenic, chez ces animaux les organes non lymphoïdes comme le coeur ou le rein ne sont pas modifiés par le traitement. L'arsenic a donc un effet préventif sur le développement de la lymphoprolifération. Le phénotype des populations lymphoïdes présentes dans cette rate et ce ganglion a été déterminé par cytométrie de flux. Comme pour les résultats du groupe 1, l'arsenic permet d'éliminer la population de celles T doubles négatives qui s'accumulent au cours du vieillissement chez la souris MRL/lpr. De plus, les organes de ces souris traités ont un poids normal, il n'y a donc ni la lymphoprolifération ni l'hépatomégalie observés chez les souris témoins. Le foie, la rate, un ganglion lymphatique, un oeil, etc...., ont été prélevés afin d'être analysés en anatomopathologie.

### Résultats du groupe 3

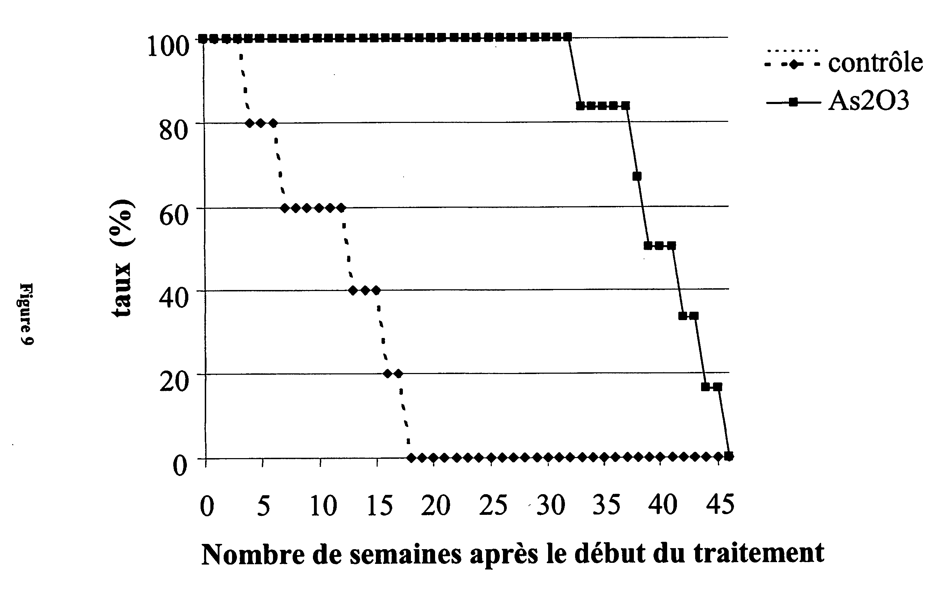

Cette étude vise à déterminer la durée de vie des animaux traités. Cette expérience a commencé à T0, des souris MRL/lpr très malades ont été traitées soit par de l'arsenic soit par le tampon de dilution de l'arsenic. En date T0+14 semaines, 100% des MRL/lpr traitées dont vivantes alors que 100% des souris MRL/lpr témoins, non traitées, sont mortes. De plus, après 35 semaines de traitement, les PRL/lpr sont encore vivantes sans aucun signe de lymphoprolifération ou de pathologies auto-immunes (figure 9)
De plus, des souris MRL+/+, irradiées de façon létale, ont été reconstituées par de la moelle osseuse de souris MRL/lpr (chimères MRL/lpr→ MRL+/+) ou par de la moelle osseuse de souris MRL+/+ (chimères MRL/+/+→ MRL+/+) comme témoin. Après reconstitution, les chimères sont traitées ou non par de l'arsenic. Alors que 100% des chimères MRL/lpr→ MRL+/+ non-traitées sont mortes d'une réaction du greffon contre l'hôte (GvH) deux semaines après la greffe, les chimères traitées par l'arsenic étaient vivantes. L'arsenic est donc efficace contre la GvH et permet d'augmenter de façon spectaculaire la survie des animaux.

### Résultats du groupe 4

Cette étude vise à déterminer l'effet dose.

Il est souhaitable de déterminer de façon très précise l'effet préventif et curatif de différentes doses d'arsenic sur l'évolution des pathologies développées par les souris MRL/lpr. Cinq groupes d'animaux (MRL/lpr et MRL+/+) ont été traités avec les concentrations suivantes : 2,5 ; 5 ; 7,5 ; 10 et 15µg/g de souris.

La dose de 15µg/g de souris s'est avérée mortelle en quelques jours pour le traitement curatif. Pour le traitement préventif, la dose est également très toxique et les animaux sont morts au bout de deux mois. La dose de 2,5µg/g de souris n'est pas suffisamment efficace pour le traitement curatif au vu des atteintes cutanées et de la taille des ganglions visible depuis l'extérieur de l'animal (et comme observé après ouverture de l'animal). Les concentrations de 5 ; 7,5 et 10µg/g entraînent une disparition complète des atteintes cutanées. L'effet curatif complet a été confirmé sur les différents organes étudiés. La dose efficace la plus faible (5µg/g pour la souris) d'arsenic est de préférence utilisée afin d'éviter tout risque de toxicité.

### Résultats du groupe 5

Cette étude vise à évaluer l'effet d'un arrêt du traitement.

Une partie des animaux du groupe 1 dont les atteintes cutanées avaient complètement disparues et dont les soeurs sacrifiées ont montré une guérison complète ont eu leur traitement arrêté de façon progressive sur deux semaines. Ces animaux présentent après trois mois d'arrêt du traitement, les pathologies cutanées caractéristiques du lupus, une réapparition de la lymphoprolifération et de l'hépatomégalie (Tableau 1). Ces résultats indiquent, que du moins pour une mutation homozygote, comme dans le cas de la souris MRL/lpr le traitement ne peut pas être interrompu de façon définitive. Dans la mesure où la mutation est hétérozygote chez l'homme, les possibilités de traitement sont accrues.
L'ensemble de ces résultats permet de confirmer l'efficacité d'une thérapie par l'arsenic de maladies auto-immunes chez l'homme. De plus l'homme du métier disposant des connaissances concernant la thérapie par l'arsenic chez l'homme d'une leucémie particulière, la Leucémie Aiguë Promyélocytaire, et grâce à l'invention désormais également les doses efficaces chez la souris, son utilisation pour le traitement de maladies auto-immunes chez l'homme telles que l'ALPS ne pose pas de problèmes. De plus ce traitement est très avantageux car il n'est pas coûteux.

## Revendications

1. Utilisation d'un composé arsenic choisi dans le groupe constitué par As₂O₃ As₄O₆, As₂S₂, As₂S₃, As₂S₅, As₄S₄, de préférence As₂O₃, pour la préparation d'un médicament pour le traitement et/ou la prévention de maladies auto-immunes.

2. Utilisation selon la revendication 1 pour le traitement d'une maladie auto-immune due à un défaut de maintien de l'homéostasie des organes lymphoïdes.

3. Utilisation selon la revendication 1 pour le traitement d'une maladie auto-immune due à un défaut de maintien de l'homéostasie du foie.

4. Utilisation selon la revendication 1 pour le traitement d'une maladie auto-immune cutanée.

5. Utilisation selon la revendication 1 pour le traitement d'une maladie auto-immune due à une apoptose défectueuse de cellules proliférantes du foie, de la peau, du système immunitaire.

6. Utilisation selon la revendication 5 pour le traitement d'une maladie auto-immune due à une apoptose défectueuse de cellules proliférantes du système immunitaire pouvant se traduire entre autre par une surexpression de FasL.

7. Utilisation selon la revendication 5 pour le traitement d'une maladie auto-immune due à une apoptose défectueuse de cellules proliférantes du système immunitaire pouvant se traduire entre autre par une production anormale de cytokines.

8. Utilisation selon la revendication 5 ou 7 pour le traitement du diabète sucré insulino-dépendant.

9. Utilisation selon la revendication 2 pour le traitement du syndrome auto-immun lymphoprolifératif (ALPS).

10. Utilisation selon la revendication 4 pour le traitement du Lupus érythémateux.

11. Utilisation selon la revendication 5 ou 7 pour le traitement de la polyarthrite rhumatoïde.

12. Utilisation selon la revendication 1 pour le traitement du syndrome de Sjögren.

13. Utilisation selon la revendication 5 ou 6 pour le traitement de l'hépatite chronique auto-immune.

14. Utilisation selon la revendication 1 pour le traitement de la réaction du greffon contre l'hôte.

15. Utilisation selon la revendication 1 pour le traitement de la maladie de Crohn.

16. Utilisation selon la revendication 1 pour le traitement de la colite ulcéreuse.

17. Utilisation selon l'une quelconque des revendications 1 à 16 **caractérisée en ce que** ledit médicament comprend une quantité efficace dudit composé arsenic.

18. Utilisation selon la revendication 17 **caractérisée en ce que** la quantité efficace administrée quotidiennement dudit composé arsenic est une dose de 3 à 10 mg/kg de poids corporel, de préférence 5 à 10 mg/kg.

19. Utilisation selon la revendication 17 ou 18 **caractérisée en ce que** ledit médicament comprend en outre un transporteur ou excipient pharmaceutiquement acceptable.

20. Utilisation selon l'une quelconque des revendications 17 à 19 **caractérisée en ce que** ledit médicament est administré par voie orale ou parentérale.

## Claims

1. Use of an arsenic compound chosen from the group consisting of As₂O₃, As₄O₆, As₂S₂, As₂S₃, As₂S₅ and As₄S₄, preferably As₂O₃, for preparing a medicinal product for treating and/or preventing autoimmune diseases.

2. Use according to Claim 1, for treating an autoimmune disease due to a deficiency in maintaining lymphoid organ homeostasis.

3. Use according to Claim 1, for treating an autoimmune disease due to a deficiency in maintaining liver homeostasis.

4. Use according to Claim 1, for treating a cutaneous autoimmune disease.

5. Use according to Claim 1, for treating an autoimmune disease due to a defective apoptosis of proliferating cells of the liver, of the skin, of the immune system.

6. Use according to Claim 5, for treating an autoimmune disease due to a defective apoptosis of proliferating cells of the immune system which can result, inter alia, in an overexpression of FasL.

7. Use according to Claim 5, for treating an autoimmune disease due to a defective apoptosis of proliferating cells of the immune system which can result, inter alia, in an abnormal production of cytokines.

8. Use according to Claim 5 or 7, for treating insulin-dependent diabetes mellitus.

9. Use according to Claim 2, for treating autoimmune lymphoproliferative syndrome (ALPS).

10. Use according to Claim 4, for treating lupus erythematosus.

11. Use according to Claim 5 or 7, for treating rheumatoid arthritis.

12. Use according to Claim 1, for treating Sjögren's syndrome.

13. Use according to Claim 5 or 6, for treating autoimmune chronic hepatitis.

14. Use according to Claim 1, for treating the graft-versus-host reaction.

15. Use according to Claim 1, for treating Crohn's disease.

16. Use according to Claim 1, for treating ulcerative colitis.

17. Use according to any one of Claims 1 to 16, **characterized in that** said medicinal product comprises an effective amount of said arsenic compound.

18. Use according to Claim 17, **characterized in that** the effective amount of said arsenic compound that is administered daily is a dose of 3 to 10 mg/kg of bodyweight, preferably 5 to 10 mg/kg.

19. Use according to Claim 17 or 18, **characterized in that** said medicinal product also comprises a pharmaceutically acceptable transporter or excipient.

20. Use according to any one of Claims 17 to 19, **characterized in that** said medicinal product is administered orally or parenterally.

## Patentansprüche

1. Verwendung einer Arsenverbindung, ausgewählt aus der Gruppe, die von As₂0₃, As₄0₆, As₂S₂, As₂S₃, As₂S₅, As₄S₄ gebildet wird, vorzugsweise As₂0₃, zur Zubereitung eines Arzneimittels zur Behandlung und/oder Vorbeugung von Autoimmunerkrankungen.

2. Verwendung nach Anspruch 1 zur Behandlung einer Autoimmunerkrankung, die auf einen Fehler bei der Aufrechterhaltung der Homöostase der lymphoiden Organe zurückzuführen ist.

3. Verwendung nach Anspruch 1 zur Behandlung einer Autoimmunerkrankung, die auf einen Fehler bei der Aufrechterhaltung der Homöostase der Leber zurückzuführen ist.

4. Verwendung nach Anspruch 1 zur Behandlung einer Autoimmunerkrankung der Haut.

5. Verwendung nach Anspruch 1 zur Behandlung einer Autoimmunerkrankung, die auf eine fehlerhafte Apoptose proliferiender Zellen der Leber, der Haut, des Immunsystems zurückzuführen ist.

6. Verwendung nach Anspruch 5 zur Behandlung einer Autoimmunerkrankung, die auf eine fehlerhafte Apoptose proliferiender Zellen des Immunsystems zurückzuführen ist, was sich unter anderem durch eine Überexpression von FasL ausdrücken kann.

7. Verwendung nach Anspruch 5 zur Behandlung einer Autoimmunerkrankung, die auf eine fehlerhafte Apoptose proliferiender Zellen des Immunsystems zurückzuführen ist, was sich unter anderem durch eine abnorme Produktion von Zytokinen ausdrücken kann.

8. Verwendung nach Anspruch 5 oder 7 zur Behandlung des insulinabhängigen Diabetes mellitus.

9. Verwendung nach Anspruch 2 zur Behandlung des autoimmunen lymphoproliferativen Syndroms (ALPS).

10. Verwendung nach Anspruch 4 zur Behandlung von Lupus erythematodes.

11. Verwendung nach Anspruch 5 oder 7 zur Behandlung der rheumatoiden Polyarthritis.

12. Verwendung nach Anspruch 1 zur Behandlung des Sjögren-Syndroms.

13. Verwendung nach Anspruch 5 oder 6 zur Behandlung der autoimmunen chronischen Hepatitis.

14. Verwendung nach Anspruch 1 zur Behandlung der Reaktion des Transplantats gegen den Wirt.

15. Verwendung nach Anspruch 1 zur Behandlung von Morbus Crohn.

16. Verwendung nach Anspruch 1 zur Behandlung von Colitis ulcerosa.

17. Verwendung nach einem der Ansprüche 1 bis 16, **dadurch gekennzeichnet, dass** das Arzneimittel eine wirksame Menge der Arsenverbindung umfasst.

18. Verwendung nach Anspruch 17, **dadurch gekennzeichnet, dass** die täglich verabreichte wirksame Menge der Arsenverbindung eine Dosis von 3 bis 10 mg/kg Körpergewicht ist, vorzugsweise 5 bis 10 mg/kg.

19. Verwendung nach Anspruch 17 oder 18, **dadurch gekennzeichnet, dass** das Arzneimittel weiterhin einen pharmazeutisch akzeptablen Transporteur oder Arzneimittelträger umfasst.

20. Verwendung nach einem der Ansprüche 17 bis 19, **dadurch gekennzeichnet, dass** besagte Arzneimittel oral oder parenteral verabreicht wird.
